# EUROPEAN PATENT APPLICATION

(11) **EP 4 635 498 A1**
(43) Date of publication of application: **22.10.2025**
(21) Application number: 23902501.8
(22) Date of filing: 29.11.2023
(51) Int. Cl.: A61K 31/522, C07D 473/32, A61K 9/20, A61K 9/48, A61K 9/14, A61P 35/00

(54) **PHARMACEUTICAL PREPARATION OF IMIDAZOLINONE COMPOUND, PREPARATION METHOD THEREFOR, AND USE THEREOF**

(30) Priority: 13.12.2022 CN 202211593096
(71) Applicant: Kangbaida (Sichuan) Biotechnology Co., Ltd., Chengdu, Sichuan 611137 (CN)
(72) Inventor: CHU, Hongzhu, Chengdu, Sichuan 611130 (CN); WANG, Hao, Chengdu, Sichuan 611130 (CN); WEI, Yonggang, Chengdu, Sichuan 611130 (CN); SUN, Yi, Chengdu, Sichuan 611130 (CN)
(74) Representative: Carpmaels & Ransford LLP
(86) International application number: PCT/CN2023/135097
(87) International publication number: WO 2024/125304

(57) **Abstract**

The present invention relates to a pharmaceutical preparation of an imidazolinone compound and a preparation method therefor. In particular, the present invention relates to a pharmaceutical preparation of a compound represented by formula I

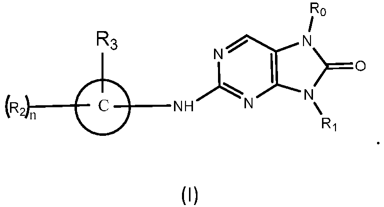

## Description

### TECHNICAL FIELD

The present invention relates to the field of pharmaceutical preparations, and in particular relates to a pharmaceutical preparation of an imidazolinone compound, a preparation method therefor and the use thereof.

### BACKGROUND ART

DNA-dependent protein kinase (DNA-PK) is a DNA-PK enzyme complex composed of a Ku70/Ku80 heterodimer and a DNA-dependent protein kinase catalytic subunit (DNA-PKcs). The enzyme complex can only be activated to perform the corresponding function thereof with the participation of DNA (George et al., 2019). As a serine/threonine protein kinase, DNA-PK is a member of the PIKK (phosphatidylinositol 3-kinase-related kinase) family. It not only plays an important role in repairing DNA double-strand breaks (DSBs) in cells and cellular DNA recombination or antibody DNA rearrangement (V(D)J recombination), but also participates in physiological processes such as chromosome modification, transcriptional regulation, and telomere maintenance.

When a DNA-PK inhibitor is used in combination with an anti-tumor therapy that causes DNA damage (e.g., IR and chemotherapeutic reagents), the therapeutic effect can be improved. Although the use of a DNA-PK inhibitor can interfere with the DNA repair function of normal cells to some extent, there are still a variety of DNA repair pathways in normal cells as a complement; in addition, facing tremendous DNA replication pressure and the lack of effective DNA repair methods, the tumor cells are thus more sensitive to a DNA-PK inhibitor, and inhibiting the DNA-PK activity of tumor cells can enhance the killing effect of other anti-tumor therapies on tumor cells.

The patent (Application No.: PCT/CN 2021/087912) describes a novel DNA-PK inhibitor having a structure represented by formula (A). It has a good inhibitory effect on DNA-PK activity and has the potential to prepare anti-tumor drugs. It is necessary to prepare a DNA-PK-inhibitor-containing preparation with a good drug dissolution rate and stability.

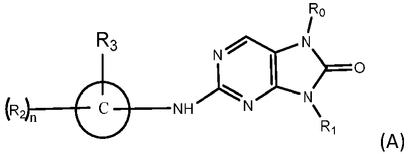

### SUMMARY OF THE INVENTION

The present invention provides a pharmaceutical preparation of an imidazolinone compound, which is convenient for patients to use and has characteristics such as stable quality and good dissolution effect.

The present invention provides a pharmaceutical preparation of an imidazolinone compound, comprising an imidazolinone compound, or a stereoisomer, solvate, prodrug, metabolite, pharmaceutically acceptable salt or co-crystal thereof, wherein the pharmaceutical preparation comprises the imidazolinone compound above with a specification of 1-1000 mg, and the imidazolinone compound is selected from a compound of formula I: 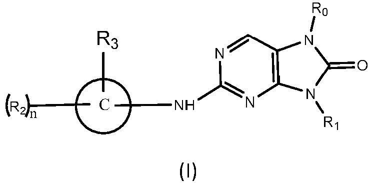 wherein 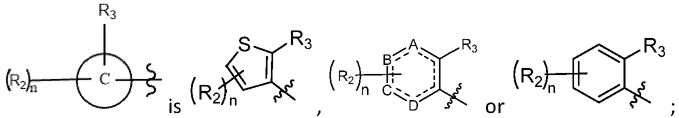
-̅ -̅ -̅ is a single bond or double bond; in 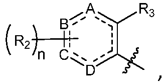 A, B, C, D are each independently C or N, and at least one of A, B, C and D is N;
R₀ is H, C₁₋₆ alkyl or cyclopropyl, wherein the C₁₋₆ alkyl is optionally further substituted with one or more substituents selected from halogen and deuterium;
R₁ is 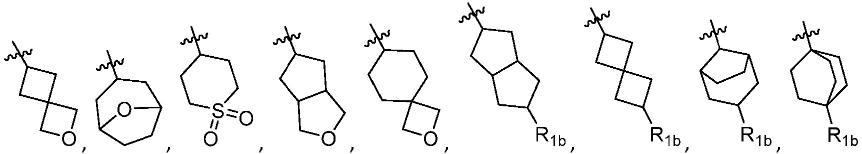 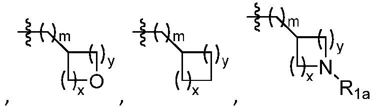 or pyridyl, and R₁ is optionally further substituted with 1 or 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R₂ is H, cyano, =O, carboxyl, -C(=O)NR₂ₐR_{2b}, C₁₋₆ alkoxy, C₁₋₆ alkyl, halogen, -S(=O)₂R₂ₐ or -C(=O)OC₁₋₆ alkyl, wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1, 2 or 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that when 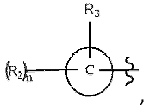 R₀, R₂, and R₃ simultaneously satisfy the following conditions, R₁ is not 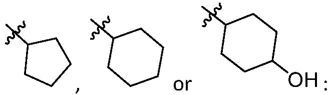 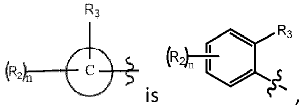 n is 1, R₀ is H or methyl, R₂ is methoxy or -S(=O)₂Me, and R₃ is methyl.

Further, in one or more examples of the present invention, in the compound of formula I described above,
R₁ is 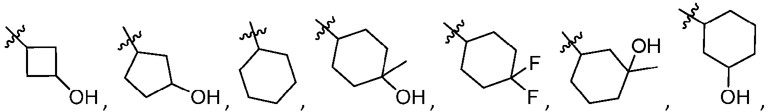 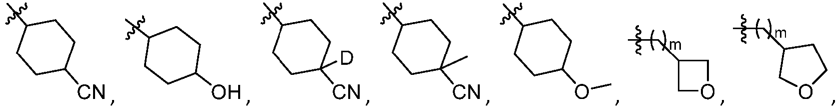 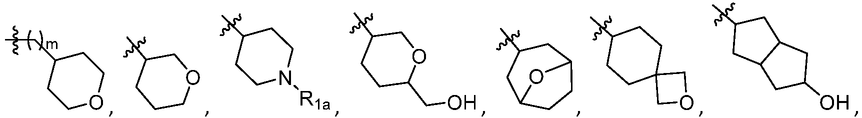 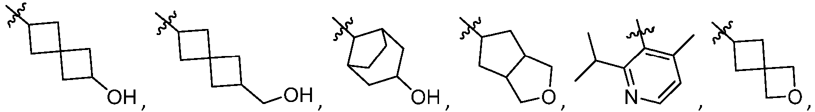 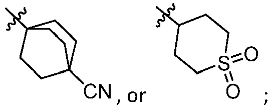
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2.

Further, in one or more examples of the present invention, in the compound of formula I, 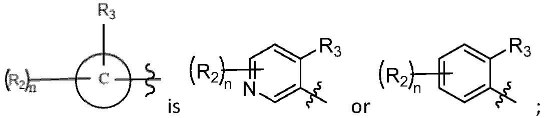
R₀ is H, C₁₋₄ alkyl or cyclopropyl, wherein the C₁₋₄ alkyl is optionally further substituted with one or more substituents selected from halogen and D;
R₁ is 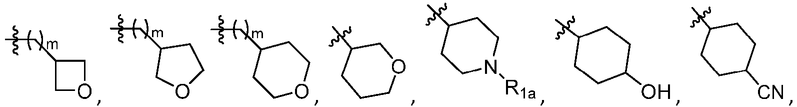 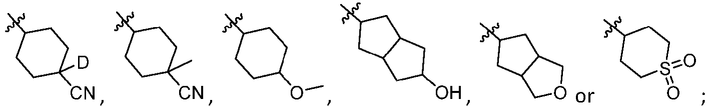
R₁ₐ is H, C₁₋₆ alkyl or -C(=O)C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D and halogen;
alternatively, R₂ₐ and R_{2b} together with the atoms to which they are attached form a 5- to 6-membered heterocyclyl, which contains 1 to 3 heteroatoms selected from N, O and S and is also optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2.

Further, in one or more examples of the present invention, the compound of formula I described above is selected from: 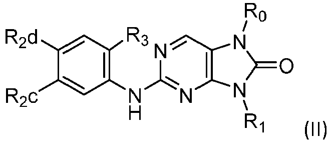 wherein
R₀ is H, C₁₋₆ alkyl or cyclopropyl, wherein the C₁₋₆ alkyl is optionally further substituted with one or more substituents selected from halogen and deuterium;
R₁ is 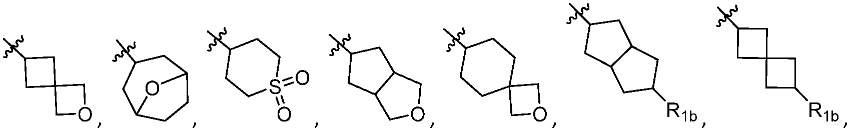 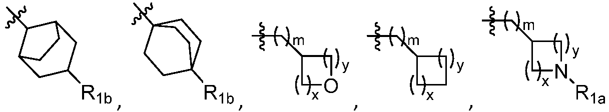 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R_{2c} is H, cyano, halogen or C₁₋₆ alkoxy;
R_{2d} is H, cyano, carboxyl, -C(=O)NR₂ₐR_{2b}, C₁₋₆ alkyl, halogen, -S(=O)₂R₂ₐ or -C(=O)OC₁₋₆ alkyl, wherein the C₁₋₆ alkyl and -C(=O)OC₁₋₆ alkyl is optionally substituted with one or more substituents selected from halogen and deuterium;
R₂ₐ and R_{2b} are H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3.

Further, in one or more examples of the present invention, the compound of formula I described above is selected from: 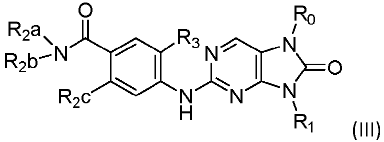 wherein
R₀ is H, C₁₋₆ alkyl or cyclopropyl, wherein the C₁₋₆ alkyl is optionally further substituted with one or more substituents selected from halogen and deuterium;
R₁ is 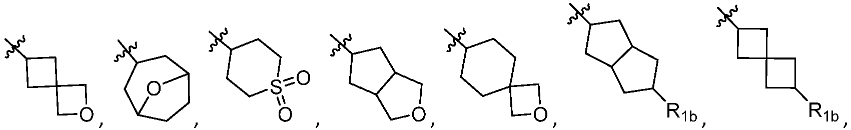 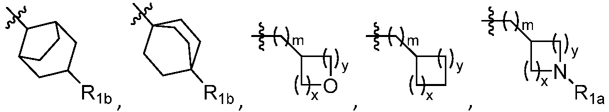 or pyridyl, and R₁ is optionally further substituted with 1 to 2 substituents selected from D, halogen, cyano, hydroxyl, C₁₋₆ alkyl and C₁₋₆ alkoxy;
R_{1b} is H, OH, cyano, or hydroxyl substituted C₁₋₆ alkyl;
R₂ₐ and R_{2b} are each independently H, C₁₋₆ alkyl, or 3- to 5-membered cycloalkyl, wherein the C₁₋₆ alkyl is optionally further substituted with one or more substituents selected from D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R_{2c} is H, cyano, halogen or C₁₋₆ alkoxy, wherein the C₁₋₆ alkoxy is optionally substituted with one or more deuterium;
R₃ is halogen or C₁₋₆ alkyl, wherein the C₁₋₆ alkyl is optionally further substituted with 1 to 3 substituents selected from D or halogen;
m is 0 or 1;
n is 0, 1 or 2; and
x and y are each independently 1, 2 or 3.

Further, in one or more examples of the present invention, the compound of formula I described above is selected from: 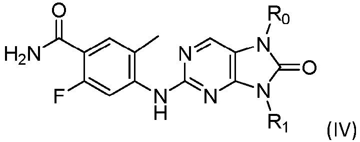 wherein
R₀ is H, C₁₋₆ alkyl or cyclopropyl, wherein the C₁₋₆ alkyl is optionally further substituted with one or more substituents selected from halogen and deuterium;
R₁ is -(CH)ₘ-4- to 7-membered carbocyclyl, -(CH)ₘ-4- to 7-membered heterocyclyl, -(CH)ₘ-8- to 12-membered bridged ring, -(CH)ₘ-7- to 12-membered spiro ring, wherein the -(CH)ₘ-4- to 7-membered carbocyclyl, -(CH)ₘ-4- to 7-membered heterocyclyl, -(CH)ₘ-8- to 12-membered bridged ring, or -(CH)ₘ-7- to 12-membered spiro ring is optionally further substituted with one or more substituents selected from hydroxy, cyano, halogen, =O, C₁₋₆ alkyl, C₁₋₆ alkoxy, and hydroxy substituted C₁₋₆ alkyl; and
m is 0 or 1.

Further, in one or more examples of the present invention, in the compound of formula I described above,
R₀ is H, C₁₋₆ alkyl or cyclopropyl, wherein the C₁₋₆ alkyl is optionally further substituted with one or more substituents selected from halogen and deuterium;
R₁ is 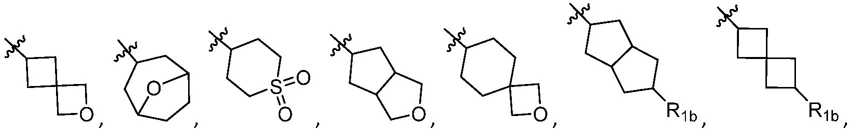 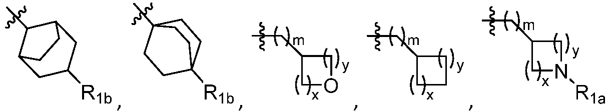 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
m is 0 or 1; and
x and y are each independently 1, 2 or 3.

Further, in one or more examples of the present invention, in the compound of formula I described above,
R₀ is C₁₋₄ alkyl, wherein the C₁₋₄ alkyl is optionally further substituted with one or more substituents selected from halogen and deuterium; and
R₁ is

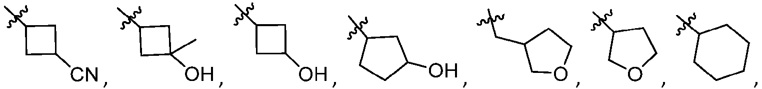

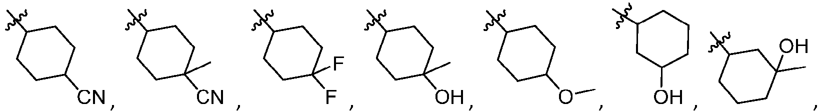

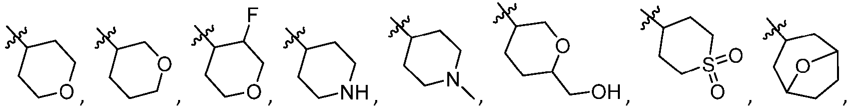

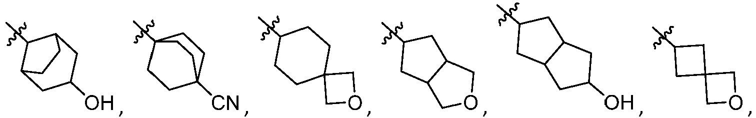

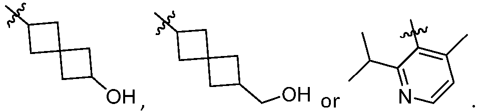

Further, in one or more examples of the present invention, the compound of formula I described above is selected from:

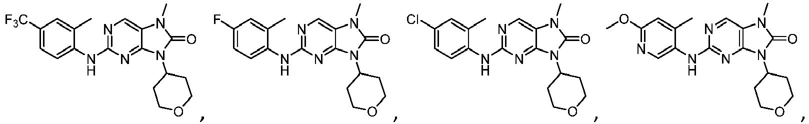

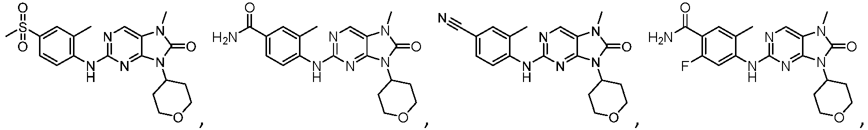

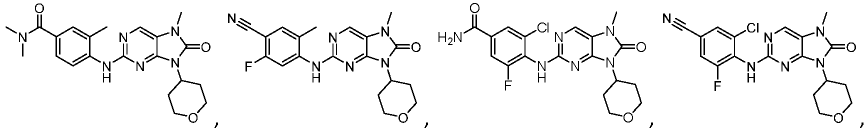

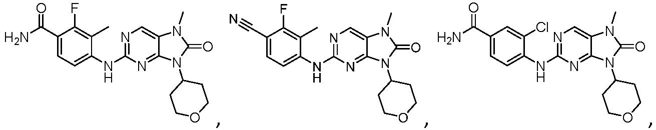

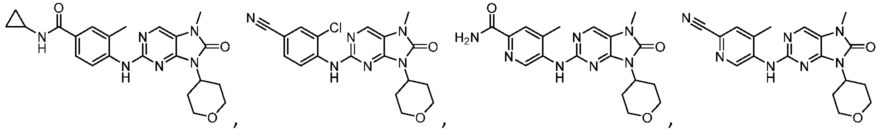

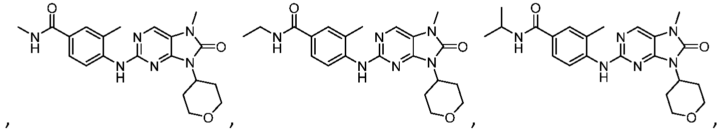

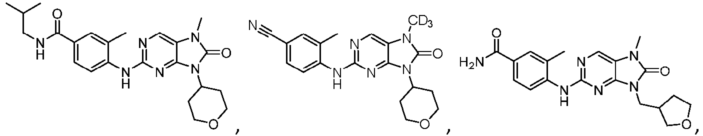

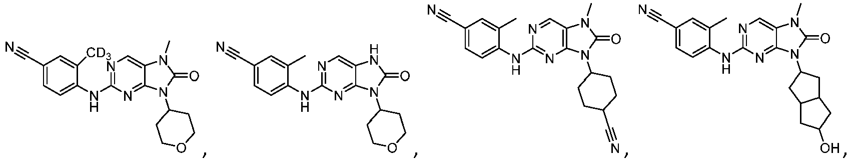

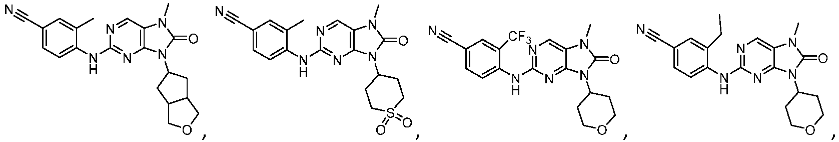

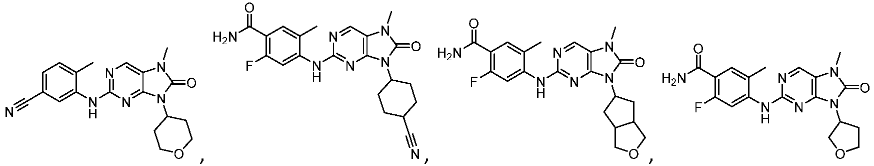

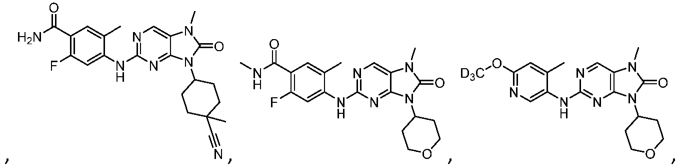

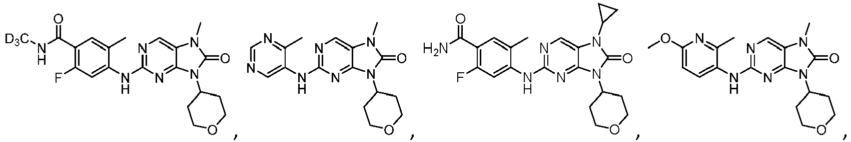

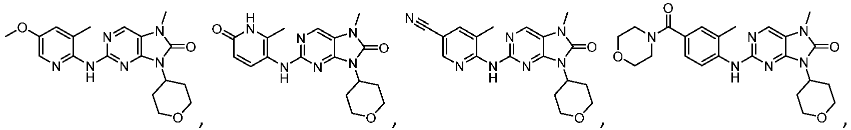

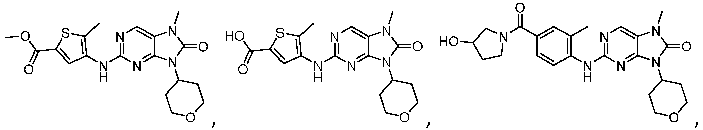

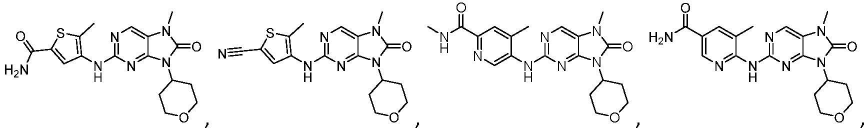

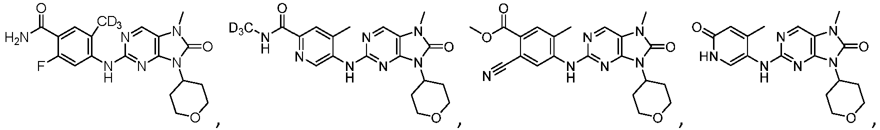

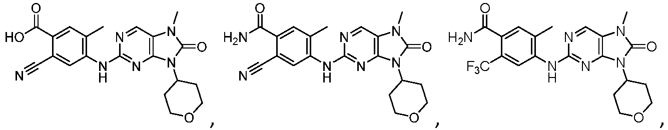

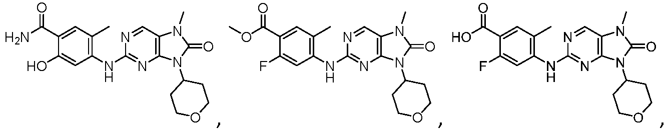

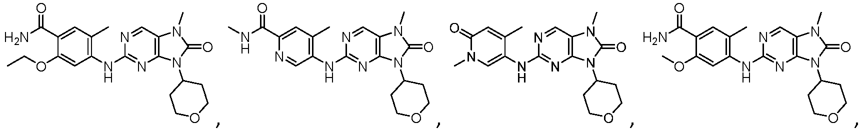

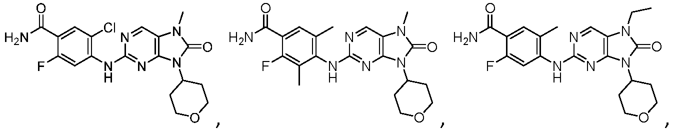

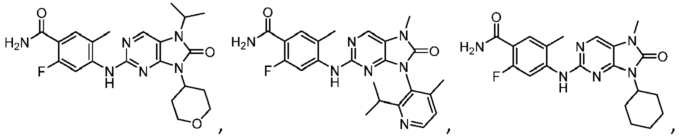

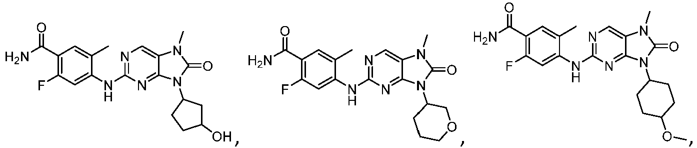

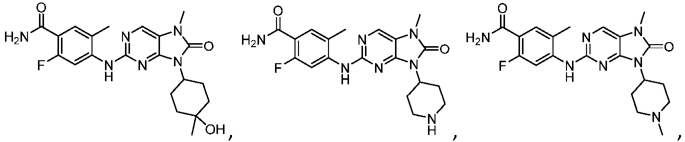

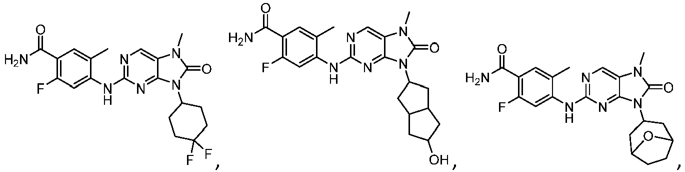

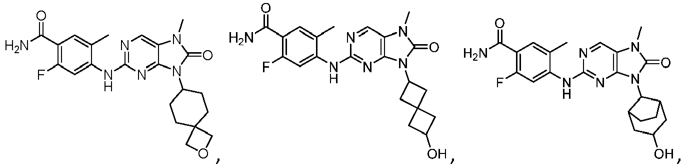

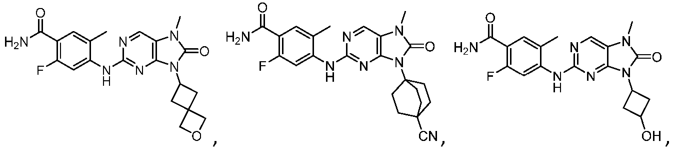

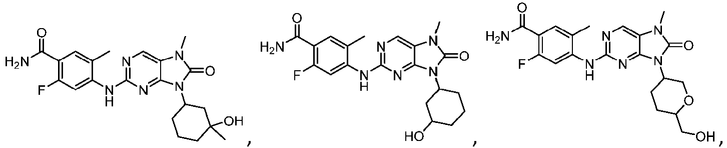

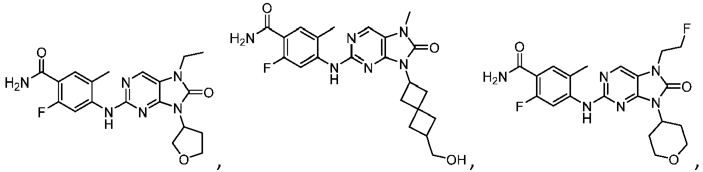

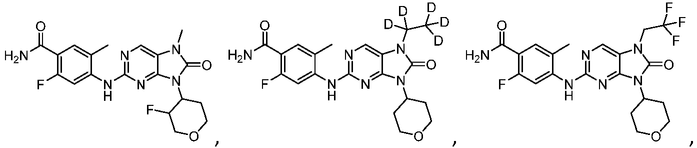

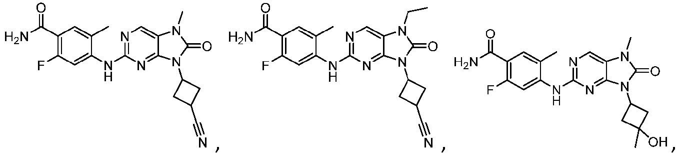

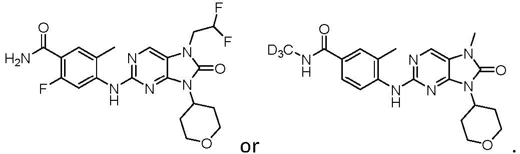

The present invention further provides a pharmaceutical preparation of an imidazolinone compound, and in one or more examples, the pharmaceutical preparation comprises a compound of formula I with a specification of 1-5 mg, 5-10 mg, 10-15 mg, 15-20 mg, 20-25 mg, 25-30 mg, 30-35 mg, 35-40 mg, 40-50 mg, 50-60 mg, 60-70 mg, 70-80 mg, 80-90 mg, 90-100 mg, 100-150 mg, 150-200 mg, 200-250 mg, 250-300 mg, 300-350 mg, 350-400 mg, 400-450 mg, 450-500 mg, 500-600 mg, 600-700 mg, 700-800 mg, 800-900 mg or 900-1000 mg.

The present invention further provides a pharmaceutical preparation of an imidazolinone compound, comprising an imidazolinone compound, or a stereoisomer, solvate, prodrug, metabolite, pharmaceutically acceptable salt or co-crystal thereof, wherein the imidazolinone compound is selected from any one or more of the compounds of formula I described above, and the content of the imidazolinone compound in the pharmaceutical preparation is 1-90 parts by weight.

Further, in one or more examples of the present invention, the content of the imidazolinone compound in the pharmaceutical preparation is 1-5 parts, 5-10 parts, 10-15 parts, 15-20 parts, 20-25 parts, 25-30 parts, 30-35 parts, 35-40 parts, 40-45 parts, 45-50 parts, 50-55 parts, 55-60 parts, 60-65 parts, 65-70 parts, 70-75 parts, 75-80 parts, 80-85 parts or 85-90 parts; and optionally further, the content of the imidazolinone compound in the pharmaceutical preparation is 27.8 parts or 41.7 parts.

The present invention further provides a pharmaceutical preparation of an imidazolinone compound, wherein the dosage form of the pharmaceutical preparation is selected from but not limited to a granule, a tablet, a capsule, a pill, or a micropellet.

The present invention further provides a pharmaceutical preparation of an imidazolinone compound, which further comprises one or more of a diluent, a disintegrant, and a glidant, and in the pharmaceutical preparation, in parts by weight, the content of the diluent is 10-85 parts; the content of the disintegrant is 3-70 parts; and the content of the glidant is 0.1-30 parts.

Further, in one or more examples of the present invention, the pharmaceutical preparation may further contain a surfactant, a wetting agent, an antioxidant, and the like.

Further, in one or more examples of the present invention, the content of the diluent in the pharmaceutical preparation is 10-15 parts, 15-20 parts, 20-25 parts, 25-30 parts, 30-35 parts, 35-40 parts, 40-45 parts, 45-50 parts, 50-55 parts, 55-60 parts, 60-65 parts, 65-70 parts, 70-75 parts, 75-80 parts, or 80-85 parts.

Further, in one or more examples of the present invention, the content of the disintegrant is 3-5 parts, 5-15 parts, 15-20 parts, 20-25 parts, 25-30 parts, 30-35 parts, 35-40 parts, 40-45 parts, 45-50 parts, 50-55 parts, 55-60 parts, 60-65 parts, or 65-70 parts.

Further, in one or more examples of the present invention, the content of the glidant is 0.1-0.5 parts, 0.5-1 parts, 1-5 parts, 5-10 parts, 10-15 parts, 15-20 parts, 20-25 parts, or 25-30 parts.

Optionally further, in one or more examples of the present invention, in the pharmaceutical preparation, the content of the diluent is 64.1 parts, 66.7 parts or 50 parts; the content of the disintegrant is 7 parts, 4.4 parts or 6.7 parts; and the content of the glidant is 1.6 parts or 1.1 parts.

Further, in one or more examples of the present invention, the diluent is selected from but not limited to one or more of sucrose, xylitol, maltitol, lactitol, hydroxypropyl cellulose, hypromellose, sorbitol, glucose, fructose, corn starch, inorganic salts, microcrystalline cellulose, starch, mannitol, lactose and pregelatinized starch, preferably one or more of microcrystalline cellulose, mannitol and pregelatinized starch.

Further, in one or more examples of the present invention, the disintegrant is selected from but not limited to one or more of cross-linked polyvinylpyrrolidone, sodium starch glycolate, calcium carboxymethyl cellulose, potato starch, microcrystalline cellulose, low-substituted hydroxypropyl cellulose, croscarmellose sodium, crospovidone, sodium carboxymethyl starch and low-substituted hydroxypropyl cellulose.

Further, in one or more examples of the present invention, the glidant is selected from but not limited to one or more of colloidal silica, silica, magnesium silicate, magnesium trisilicate, polysilicate, hydrated silica, calcium phosphate, and talc.

The present invention further provides a preparation method for the pharmaceutical preparation of an imidazolinone compound, the preparation method mainly including the following steps:
preparing a mixture of an imidazolinone compound, a disintegrant, and a diluent; and mixing a glidant with the mixture and then preparing same into a tablet, a capsule, or a granule.

Further, in one or more examples of the present invention, after mixing a glidant with the mixture, the resulting mixture is placed in a granulator for granulation and then prepared into a tablet, a capsule, or a granule;
or further, in one or more examples of the present invention,
the mixture comprises 50-80 wt% of the prescription amount of a diluent; the mixture is placed in a mixer for mixing and then placed in a granulator for granulation to obtain mixture particles; and a glidant and the remaining prescription amount of the diluent are added to the mixture particles, and mixed in a mixer and then prepared into a tablet, a capsule, or a granule.

The present invention further provides the use of the pharmaceutical preparation of an imidazolinone compound in the preparation of a drug for the treatment and/or prevention of a cancer.

In one or more examples of the present invention, there is provided the use of the pharmaceutical preparation of an imidazolinone compound in the preparation of a drug for the treatment and/or prevention of a cancer by administering to a subject a drug comprising the pharmaceutical preparation described above; optionally further, the use involves administering the drug orally; and optionally further, the drug is administered orally 1-4 times per day.

The present invention further provides a pharmaceutical preparation of an imidazolinone compound, comprising an imidazolinone compound, or a stereoisomer, solvate, prodrug, metabolite, pharmaceutically acceptable salt or co-crystal thereof, wherein the imidazolinone compound is selected from one or more compounds of formula I described above, the compound of formula I is present in an amount to provide a daily dose of 1-1000 mg, and the daily dose is a single-dose or multi-dose.

Further, in one or more examples of the present invention, the imidazolinone compound is present in an amount to provide a daily dose of 1-10 mg, 10-20 mg, 20-50 mg, 50-100 mg, 100-150 mg, 150-200 mg, 200-250 mg, 250-300 mg, 300-350 mg, 350-400 mg, 400-450 mg, 450-500 mg, 500-600 mg, 600-700 mg, 700-800 mg, 800-900 mg or 900-1000 mg.

In one or more examples of the present invention, there is provided a pharmaceutical preparation of an imidazolinone compound, comprising an imidazolinone compound, or a stereoisomer, solvate, prodrug, metabolite, pharmaceutically acceptable salt or co-crystal thereof, wherein the imidazolinone compound is selected from any one or more compounds of formula I described above, and the compound of formula I is present in an amount to provide a daily dose of 1-1000 mg/60 kg.

The present invention further provides a method for preventing and/or treating cancer, comprising administering to a subject a therapeutically effective dose of any pharmaceutical preparation of an imidazolinone compound described above.

In one or more examples of the present invention, the pharmaceutical preparation comprises an imidazolinone compound, or a stereoisomer, solvate, prodrug, metabolite, pharmaceutically acceptable salt or co-crystal thereof, wherein the imidazolinone compound is selected from any one or more compounds of formula I described above. Further, the compound of formula I is present in an amount to provide a daily dose of 1-1000 mg; and still further, the compound of formula I is present in an amount to provide a daily dose of 1-1000 mg/60 kg.

Unless stated to the contrary, the terms used in the description and claims have the following meanings.

The "pharmaceutically acceptable salt" refers to a salt that is safe, non-toxic, and neither biologically nor otherwise undesirable, is pharmaceutically acceptable for veterinary use and human pharmaceutical use, and has the desired pharmacological activity.

The "prodrug" refers to a compound that can be converted into the compound of the present invention with the biological activity by metabolism *in vivo.*

The "stereoisomer" refers to an isomer produced as a result of different spatial arrangement of atoms in molecules, including cis-trans isomers, enantiomers and conformational isomers.

"Optional" or "optionally" or "alternative" or "alternatively" or "further" means that the events or conditions subsequently described may but not necessarily occur, and the description includes the case where the events or conditions occur and do not occur. For example, "heterocyclyl alternatively substituted with alkyl" means that the alkyl may but not necessarily exist, and the description includes the case where the heterocyclyl is substituted with alkyl and the case where the heterocyclyl is not substituted with alkyl.

The "effective dose" refers to an amount that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

It can be understood that the numerical values described and claimed in the present invention are approximate. Variations in values may be attributed to device calibration, device errors, purity of substances, crystal size, sample size and other factors.

Without departing from the scope and spirit of the present invention, it will be apparent to those skilled in the art to make various improvements and changes to the present invention upon consideration of the specification and the operation content in the examples of the present invention.

### DETAILED DESCRIPTION OF EMBODIMENTS

The implementation process and beneficial effects of the present invention are described in detail below by way of specific examples, which are intended to help readers better understand the essence and characteristics of the present invention but not to limit the scope of implementation of the present invention.

If there is no special instruction in the examples, the solution refers to an aqueous solution.

The known starting materials of the present invention can be synthesized by or according to methods known in the art, or can be purchased from Titan Technology Co., Ltd., Energy Chemical Co., Ltd., Shanghai Demo Co., Ltd., Chengdu Kelong Chemical Co., Ltd., Accela ChemBio Co., Ltd., J&K Scientific Co., Ltd., etc.

The compound of general formula (I) or the specific structure thereof can be obtained by the method in patent WO 2021209055, which is incorporated herein by reference in its entirety.

The active pharmaceutical ingredient, i.e., compound 62, is prepared by the method below:

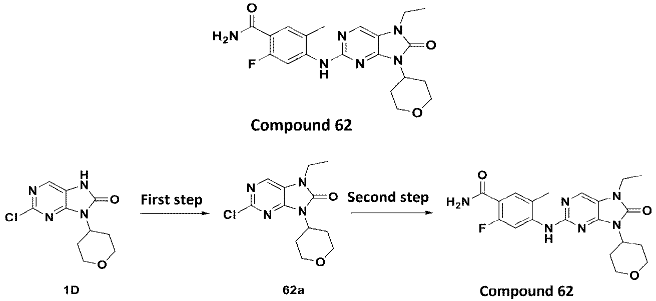

### First step:

### 2-Chloro-7-ethyl-9-(tetrahydro-2H-pyran-4-yl)-7,9-dihydro-8H-purin-8-one (62a)

2-Chloro-9-(tetrahydro-2H-pyran-4-yl)-7,9-dihydro-8H-purin-8-one **1D** (400 mg, 1.57 mmol) is dissolved in N,N-dimethylformamide (8 mL), and cesium carbonate (511 mg, 1.57 mmol) and iodoethane (293 mg, 1.88 mmol) are added at 0°C for reaction with stirring for 1 hour. The reaction is monitored using TLC until the reaction is complete. 10 mL water is then added, followed by extraction with ethyl acetate three times. The organic phase is dried using anhydrous sodium sulfate and concentrated, followed by rotary evaporation to remove the organic solvent, so as to afford the title compound 2-chloro-7-ethyl-9-(tetrahydro-2H-pyran-4-yl)-7,9-dihydro-8H-purin-8-one **62a** (white solid, 290 mg, yield: 65.32%).

¹H NMR (400 MHz, DMSO-d6) δ 8.44 (s, 1H), 4.50 -4.41 (m, 1H), 3.99 -3.95 (m, 2H), 3.89 (q, 2H), 3.45 (t, 2H), 2.46-2.41 (m, 2H), 1.71-1.67 (m, 2H), 1.25 (t, 3H).

LC-MS m/z(ESI) = **283.10** [M+1].

### Second step:

### 4-((7-Ethyl-8-oxo-9-(tetrahydro-2H-pyran-4-yl)-8,9-dihydro-7H-purin-2-yl)amino)-2-fluoro-5-methylbenzamide (compound 62)

2-Chloro-7-ethyl-9-(tetrahydro-2H-pyran-4-yl)-7,9-dihydro-8H-purin-8-one **62a** (150 mg, 0.53 mmol), 4-amino-2-fluoro-5-methylbenzamide **(intermediate 2,** 350 mg, 2.12 mmol), cesium carbonate (690 mg, 2.12 mmol), and methanesulfonato(2-dicyclohexylphosphino-3,6-dimethoxy-2',4',6'-triisopropyl-1,1'-biphenyl)(2'-amino-1,1'-biphenyl-2-yl)palladium(II) (72 mg, 0.08 mmol) are dissolved in 1,4-dioxane (5 mL) for reaction at 110°C with stirring for 4 hours under the protection of nitrogen. The reaction is monitored using TLC until the reaction is complete. The concentrated reaction solution is separated and purified via silica gel column chromatography (dichloromethane/methanol (v/v) = 20/1), followed by Pre-HPLC to afford the title compound 4-((7-ethyl-8-oxo-9-(tetrahydro-2H-pyran-4-yl)-8,9-dihydro-7H-purin-2-yl)amino)-2-fluoro-5-methylbenzamide **(compound 62,** white solid, 38 mg, yield: 17.28%).

¹H NMR (400 MHz, DMSO-d6) δ 8.53 (s, 1H), 8.25 (s, 1H), 7.89 (d, 1H), 7.55 (d, 1H), 7.42 (d, 2H), 4.48 - 4.40 (m, 1H), 3.98 (dd, 2H), 3.85 (q, 2H), 3.43 (t, 2H), 2.58 - 2.54 (m, 2H), 2.30 (s, 3H) 1.71 - 1.68 (m, 2H), 1.25 (t, 3H).

LC-MS m/z(ESI)= **415.20** [M+1].

### Examples 1-4

**Table 1**

| Raw/auxiliary material | Weight of component (mg/unit preparation) | | | | Percentage (%) |
|---|---|---|---|---|---|
| | Example 1 | Example 2 | Example 3 | Example 4 | |
| Compound 62 | 25 | 25 | 25 | 25 | 41.7 |
| Microcrystalline cellulose PH102 | 30 | - | - | - | 50.0 |
| Microcrystalline cellulose PH302 | - | 30 | - | - | 50.0 |
| Mannitol | - | - | 30 | - | 50.0 |
| Pregelatinized starch | - | - | - | 30 | 50.0 |
| Croscarmellose sodium | 4 | 4 | 4 | 4 | 6.7 |
| Colloidal silica | 1 | 1 | 1 | 1 | 1.6 |

### Preparation method:

1) prescription amounts of compound 62, microcrystalline cellulose (PH102 and PH302), mannitol, pregelatinized starch, croscarmellose sodium, and colloidal silica were weighed and respectively passed through a 60 mesh sieve for later use;
2) a prescription amount of microcrystalline cellulose PH102 (or microcrystalline cellulose PH302, mannitol, and pregelatinized starch) and a prescription amount of croscarmellose sodium were weighed respectively, mixed in an incremental manner and passed through a 60 mesh sieve twice to obtain a mixture 1;
3) a prescription amount of compound 62 was added to mixture 1, passed through a 60 mesh sieve and mixed until uniform to obtain a mixture 2;
4) a prescription amount of colloidal silica was added to mixture 2, passed through a 60 mesh sieve and mixed until uniform to obtain an intermediate powder; and
5) about 60 mg of the intermediate powder was filled into a hollow gelatin capsule to obtain a capsule with a specification of 25 mg.

### Examples 5-8

**Table 2**

| Raw/auxiliary material | Weight of component (mg/unit preparation) | | | | Percentage (%) |
|---|---|---|---|---|---|
| | Example 5 | Example 6 | Example 7 | Example 8 | |
| Compound 62 | 25 | 25 | 25 | 25 | 27.8 |
| Microcrystalline cellulose PH102 | 60 | 60 | 60 | 60 | 66.7 |
| Croscarmellose sodium | 4 | - | - | - | 4.4 |
| Crospovidone | - | 4 | - | - | 4.4 |
| Sodium carboxymethyl starch | - | - | 4 | - | 4.4 |
| Low-substituted hydroxypropylcellulose | - | - | - | 4 | 4.4 |
| Colloidal silica | 1 | 1 | 1 | 1 | 1.1 |

### Preparation method:

1) prescription amounts of compound 62, microcrystalline cellulose, croscarmellose sodium, crospovidone, sodium carboxymethyl starch, low-substituted hydroxypropylcellulose, and colloidal silica were weighed and respectively passed through a 60 mesh sieve for later use;
2) a prescription amount of microcrystalline cellulose, and a prescription amount of croscarmellose sodium (or crospovidone, sodium carboxymethyl starch, and low-substituted hydroxypropylcellulose) were weighed respectively, mixed in an incremental manner and passed through a 60 mesh sieve twice to obtain a mixture 1;
3) a prescription amount of compound 62 was added to mixture 1 and passed through a 60 mesh sieve; and a prescription amount of colloidal silica was added, passed through a 60 mesh sieve and mixed until uniform to obtain a mixture 2;
4) the mixture 2 was placed in a dry granulator for granulation, with a roller speed set at 3.00-8.00 Hz, a granulation speed set at 20.00-30.00 Hz, and a hydraulic pressure set at 30-75 kg/cm³, thus obtaining intermediate particles; and
5) about 90 mg of the intermediate particles were filled into a hollow gelatin capsule to obtain a capsule with a specification of 25 mg.

### Examples 9 and 10

**Table 3**

| Raw/auxiliary material | Weight of component (mg/unit preparation) | | Percentage (%) |
|---|---|---|---|
| | Example 9 | Example 10 | |
| Compound 62 | 25 | 100 | 27.8 |
| Microcrystalline cellulose Type 102 | 57.7 | 230.8 | 64.1 |
| Croscarmellose sodium | 6.3 | 25.2 | 7.0 |
| Colloidal silica | 1 | 4 | 1.1 |

### Preparation method:

1) prescription amounts of compound 62, microcrystalline cellulose, croscarmellose sodium, and colloidal silica were weighed and respectively passed through a 60 mesh sieve for later use;
2) a prescription amount of compound 62, and 80% of the prescription amount of microcrystalline cellulose and croscarmellose sodium were weighed and passed through a 60 mesh sieve twice to obtain a mixture 1;
3) the mixture 1 was placed in a three-dimensional mixer and mixed for 20 min, with a mixing frequency set at 40 ± 5 Hz, thus obtaining a mixture 2;
4) the mixture 2 was placed in a dry granulator for granulation, with a roller speed set at 3.00-8.00 Hz, a granulation speed set at 20.00-30.00 Hz, and a hydraulic pressure set at 30-75 kg/cm³, thus obtaining particles of mixture 3;
5) about 20% of the prescription amount of microcrystalline cellulose and colloidal silica were added to mixture 3, and mixed in a three-dimensional mixer for 20 min, with a mixing frequency set at 40 ± 5 Hz, thus obtaining an intermediate; and
6) Example 9: about 90 mg of the intermediate particles were filled into a hollow gelatin capsule to obtain a capsule with a specification of 25 mg.

Example 10: about 360 mg of the intermediate particles were filled into a hollow gelatin capsule to obtain a capsule with a specification of 100 mg.

### Dissolution test 1:

According to the dissolution rate test method (Chinese Pharmacopoeia 2020 Edition Volume IV 0931, Method 2), 900 ml of a dissolution medium of a pH 6.8 phosphate buffer was added to the samples of Examples 1, 2, 3, 4, respectively. The rotation speed was 75 revolutions per minute, and sampling was conducted at 10, 15, 20, 30, 45, 60 and 90 minutes to determine the dissolution rate. The results are show in Table 4:

**Table 4 Dissolution rate results**

| Time | Cumulative dissolution rate (%) | | | | | | |
|---|---|---|---|---|---|---|---|
| | 10 min | 15 min | 20 min | 30 min | 45 min | 60 min | 90 min |
| Example 1 | 55.02 | 74.77 | 82.20 | 87.76 | 90.31 | 91.20 | 91.86 |
| Example 2 | 51.14 | 72.66 | 81.51 | 87.28 | 90.08 | 90.92 | 91.64 |
| Example 3 | 64.64 | 84.29 | 91.63 | 97.27 | 101.03 | 100.05 | 100.81 |
| Example 4 | 60.15 | 76.71 | 84.89 | 91.80 | 95.87 | 97.30 | 98.63 |

As can be seen from the dissolution rate results above, the capsules prepared using various types of diluents, as illustrated in the examples, all have a dissolution rate greater than 80% after 20 min, and their dissolution behavior is substantially identical.

### Dissolution test 2

According to the dissolution rate test method (Chinese Pharmacopoeia 2020 Edition Volume IV 0931, Method 2), 900 ml of a dissolution medium of a pH 6.8 phosphate buffer was added to the samples of Examples 5, 6, 7, 8, respectively. The rotation speed was 75 revolutions per minute, and sampling was conducted at 10, 15, 20, 30, 45, and 60 minutes to determine the dissolution rate. The results are show in Table 5:

**Table 5 Dissolution rate results**

| Time | Cumulative dissolution rate (%) | | | | | |
|---|---|---|---|---|---|---|
| | 10 min | 15 min | 20 min | 30 min | 45 min | 60 min |
| Example 5 | 56.26 | 68.91 | 74.23 | 79.33 | 82.66 | 84.36 |
| Example 6 | 49.80 | 65.01 | 71.27 | 78.07 | 82.80 | 85.65 |
| Example 7 | 53.75 | 66.53 | 72.59 | 78.37 | 83.73 | 85.96 |
| Example 8 | 48.11 | 60.84 | 66.97 | 73.24 | 78.22 | 81.22 |

As can be seen from the dissolution rate results above, the dissolution behavior of the capsules is substantially not affected by different types of disintegrants used according to the examples, and the capsules all have a dissolution rate greater than 70% after 30 min.

### Dissolution test 3

According to the dissolution rate test method (Chinese Pharmacopoeia 2020 Edition Volume IV 0931, Method 2), 900 ml of dissolution mediums of 1) a pH 1.0 hydrochloric acid solution, 2) a pH 4.5 acetate buffer, and 3) a pH 6.8 phosphate buffer were respectively added to the sample of Examples 9. The rotation speed was 75 revolutions per minute, and sampling was conducted at 15, 30, and 60 minutes to determine the dissolution rate. The results are show in Table 6:

**Table 6 Dissolution rate results**

| Example 9 | Cumulative dissolution rate (%) | | |
|---|---|---|---|
| Dissolution medium | pH 1.0 hydrochloric acid solution | pH 4.5 acetate buffer | pH 6.8 phosphate buffer |
| 15 min | 93.47 | 67.61 | 69.22 |
| 30 min | 95.17 | 77.04 | 83.06 |
| 60 min | 96.08 | 81.84 | 90.95 |

As can be seen from the dissolution rate results above, the samples of Example 9 at various pH environments all have good dissolution rate.

### Investigation by stability test

According to the requirements under Chinese Pharmacopoeia (2015 Edition, Volume IV) 9001 "Guidelines for the Stability Testing of Drug Substances and Preparations", the samples of Example 9 and Example 10 were investigated by influencing factor tests. The experimental results are shown in Table 7 and Table 8. The test conditions were as follows:

the samples were placed in a clean open watch glass and exposed to conditions of a high-temperature test (60 ± 2°C), a high-humidity test (RH 92.5%), and a light test (4500 ± 500 Lx). Sampling was conducted for testing at 10 days and 30 days, respectively.

**Table 7 Results of influencing factor tests of sample of Example 9**

| Investigation item | Day 0 | High-temperature test 60 ± 2°C | | High-humidity test RH 92.5% | | Light test 4500 Lx ± 500 Lx | |
|---|---|---|---|---|---|---|---|
| | | 10 days | 30 days | 10 days | 30 days | 10 days | 30 days |
| Characters | Off-white powder | Off-white powder | Off-white powder | Off-white powder | Off-white powder | Off-white powder | Off-white powder |
| Total amount of impurities (%) | 0.050 | 0.051 | 0.051 | 0.050 | 0.052 | 0.050 | 0.050 |
| Content (%) | 100.9 | 96.6 | 96.6 | 100.2 | 99.4 | 98.9 | 99.3 |

**Table 8 Results of influencing factor tests of sample of Example 10**

| Investigation item | Day 0 | High-temperature test 60 ± 2°C | | High-humidity test RH 92.5% | | Light test 4500 Lx ± 500 Lx | |
|---|---|---|---|---|---|---|---|
| | | 10 days | 30 days | 10 days | 30 days | 10 days | 30 days |
| Characters | Off-white powder | Off-white powder | Off-white powder | Off-white powder | Off-white powder | Off-white powder | Off-white powder |
| Total amount of impurities (%) | 0.050 | 0.051 | 0.050 | 0.049 | 0.050 | 0.051 | 0.049 |
| Content (%) | 98.23 | 99.55 | 97.49 | 99.20 | 97.45 | 98.99 | 98.55 |

Conclusion: After the investigation by influencing factor tests, it is found that the indicators of the samples of Examples 9 and 10 do not change significantly compared to day 0 and are all qualified, indicating that the composition of compound 62 involved in the present invention has stable quality.

## Claims

1. A pharmaceutical preparation of an imidazolinone compound, comprising an imidazolinone compound, or a stereoisomer, solvate, prodrug, metabolite, pharmaceutically acceptable salt or co-crystal thereof, wherein the pharmaceutical preparation comprises the imidazolinone compound with a specification of 1-1000 mg, and the imidazolinone compound is selected from a compound of formula I: 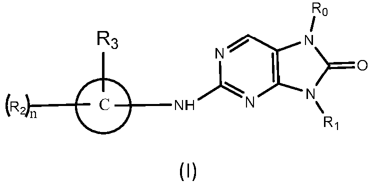 wherein 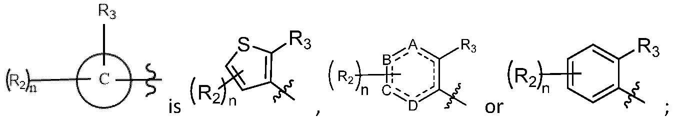
-̅ -̅ -̅ is a single bond or double bond; in 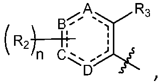 A, B, C, D are each independently C or N, and at least one of A, B, C and D is N;
R₀ is H, C₁₋₆ alkyl or cyclopropyl, wherein the C₁₋₆ alkyl is optionally further substituted with one or more substituents selected from halogen and deuterium;
R₁ is 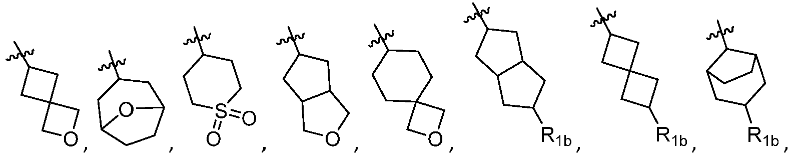 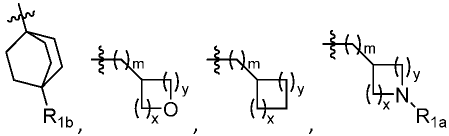 or pyridyl, and R₁ is optionally further substituted with 1 or 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R₂ is H, cyano, =O, carboxyl, -C(=O)NR₂ₐR_{2b}, C₁₋₆ alkoxy, C₁₋₆ alkyl, halogen, -S(=O)₂R₂ₐ or - C(=O)OC₁₋₆ alkyl, wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1, 2 or 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 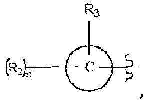 R₀, R₂, and R₃ simultaneously satisfy the following conditions, R₁ is not 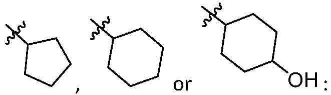 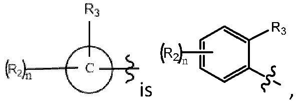 n is 1, R₀ is H or methyl, R₂ is methoxy or -S(=O)₂Me, and R₃ is methyl.

2. The pharmaceutical preparation according to claim 1, wherein in the compound of formula I,
R₁ is 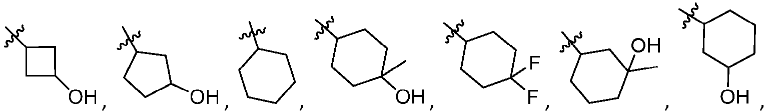 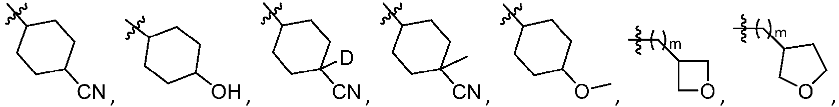 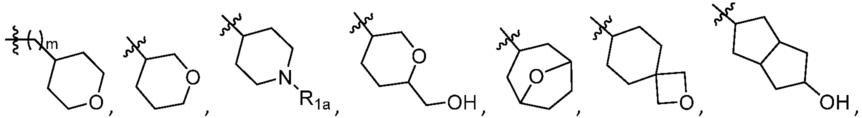 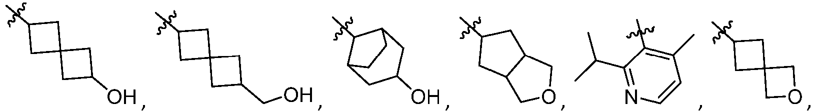 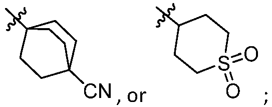
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from OH and halogen; R₃ is halogen or C₁₋₆ alkyl, wherein the C₁₋₆ alkyl is optionally further substituted with 1 to 3 substituents selected from D and halogen;
m is 0 or 1; and
n is 0, 1 or 2.

3. The pharmaceutical preparation according to claim 1, wherein in the compound of formula I, 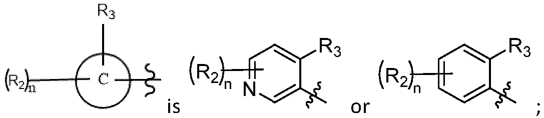
R₀ is H, C₁₋₄ alkyl or cyclopropyl, wherein the C₁₋₄ alkyl is optionally further substituted with one or more substituents selected from halogen and D;
R₁ is 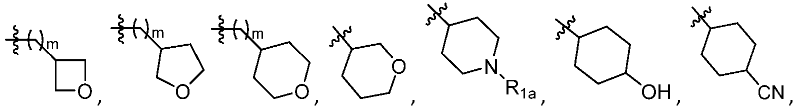 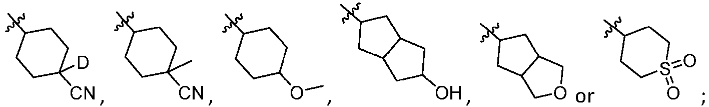
R₁ₐ is H, C₁₋₆ alkyl or -C(=O)C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D and halogen;
alternatively, R₂ₐ and R_{2b} together with the atoms to which they are attached form a 5- to 6-membered heterocyclyl, which contains 1 to 3 heteroatoms selected from N, O and S and is also optionally further substituted with one or more substituents selected from OH and halogen; R₃ is halogen or C₁₋₆ alkyl, wherein the C₁₋₆ alkyl is optionally further substituted with 1 to 3 substituents selected from D and halogen;
m is 0 or 1; and
n is 0, 1 or 2.

4. The pharmaceutical preparation according to claim 1, wherein the compound of formula I is selected from: 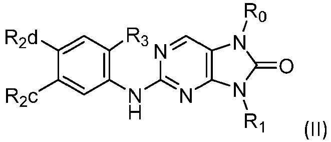 wherein
R₀ is H, C₁₋₆ alkyl or cyclopropyl, wherein the C₁₋₆ alkyl is optionally further substituted with one or more substituents selected from halogen and deuterium;
R₁ is 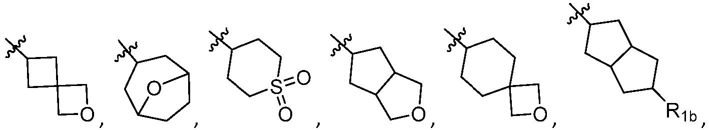 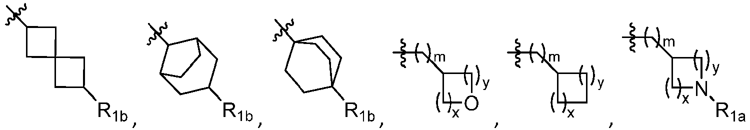 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R_{2c} is H, cyano, halogen or C₁₋₆ alkoxy;
R_{2d} is H, cyano, carboxyl, -C(=O)NR₂ₐR_{2b}, C₁₋₆ alkyl, halogen, -S(=O)₂R₂ₐ or -C(=O)OC₁₋₆ alkyl, wherein the C₁₋₆ alkyl and -C(=O)OC₁₋₆ alkyl is optionally substituted with one or more substituents selected from halogen and deuterium;
R₂ₐ and R_{2b} are H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3.

5. The pharmaceutical preparation according to claim 1, wherein the compound of formula I is selected from: 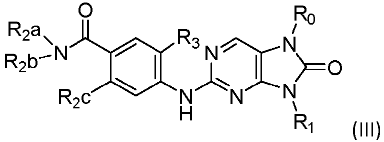 wherein
R₀ is H, C₁₋₆ alkyl or cyclopropyl, wherein the C₁₋₆ alkyl is optionally further substituted with one or more substituents selected from halogen and deuterium;
R₁ is 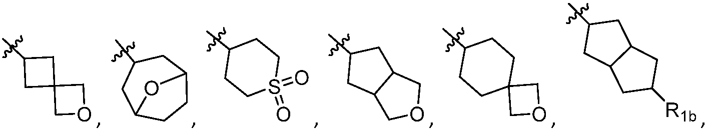 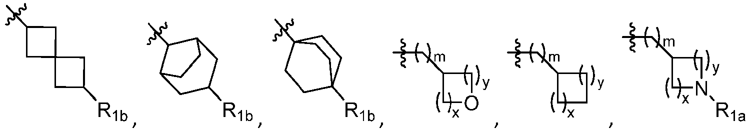 or pyridyl, and R₁ is optionally further substituted with 1 to 2 substituents selected from D, halogen, cyano, hydroxyl, C₁₋₆ alkyl and C₁₋₆ alkoxy;
R_{1b} is H, OH, cyano, or hydroxyl substituted C₁₋₆ alkyl;
R₂ₐ and R_{2b} are each independently H, C₁₋₆ alkyl, or 3- to 5-membered cycloalkyl, wherein the C₁₋₆ alkyl is optionally further substituted with one or more substituents selected from D or halogen; alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R_{2c} is H, cyano, halogen or C₁₋₆ alkoxy, wherein the C₁₋₆ alkoxy is optionally substituted with one or more deuterium;
R₃ is halogen or C₁₋₆ alkyl, wherein the C₁₋₆ alkyl is optionally further substituted with 1 to 3 substituents selected from D or halogen;
m is 0 or 1;
n is 0, 1 or 2; and
x and y are each independently 1, 2 or 3.

6. The pharmaceutical preparation according to claim 1, wherein the compound of formula I is selected from: 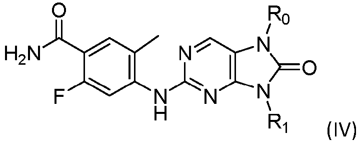 wherein
R₀ is H, C₁₋₆ alkyl or cyclopropyl, wherein the C₁₋₆ alkyl is optionally further substituted with one or more substituents selected from halogen and deuterium;
R₁ is -(CH)ₘ-4- to 7-membered carbocyclyl, -(CH)ₘ-4- to 7-membered heterocyclyl, -(CH)ₘ-8- to 12-membered bridged ring, -(CH)ₘ-7- to 12-membered spiro ring, wherein the -(CH)ₘ-4- to 7-membered carbocyclyl, -(CH)ₘ-4- to 7-membered heterocyclyl, -(CH)ₘ-8- to 12-membered bridged ring, or -(CH)ₘ-7- to 12-membered spiro ring is optionally further substituted with one or more substituents selected from hydroxy, cyano, halogen, =O, C₁₋₆ alkyl, C₁₋₆ alkoxy, and hydroxy substituted C₁₋₆ alkyl; and
m is 0 or 1.

7. The pharmaceutical preparation according to claim 6, wherein in the compound of formula I, R₀ is H, C₁₋₆ alkyl or cyclopropyl, wherein the C₁₋₆ alkyl is optionally further substituted with one or more substituents selected from halogen and deuterium;
R₁ is 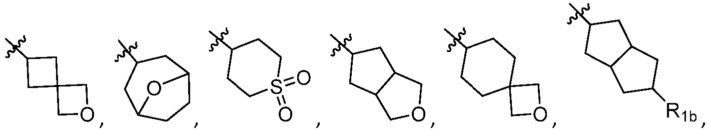 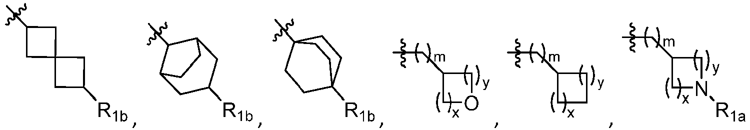 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
m is 0 or 1; and
x and y are each independently 1, 2 or 3.

8. The pharmaceutical preparation according to claim 7, wherein in the compound of formula I, R₀ is C₁₋₄ alkyl, wherein the C₁₋₄ alkyl is optionally further substituted with one or more substituents selected from halogen and deuterium; and
R₁ is

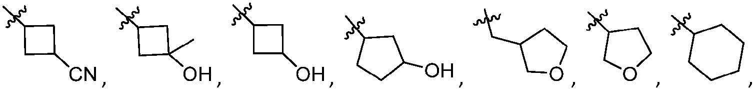

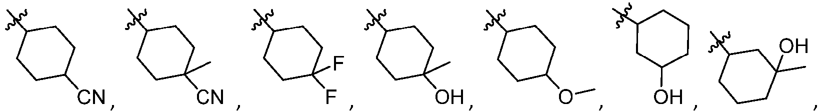

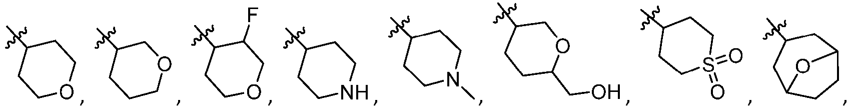

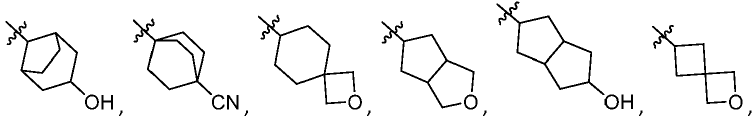

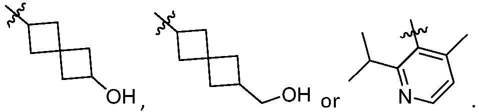

9. The pharmaceutical preparation according to claim 1, wherein the compound of formula I is selected from:

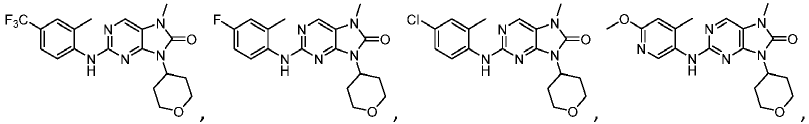

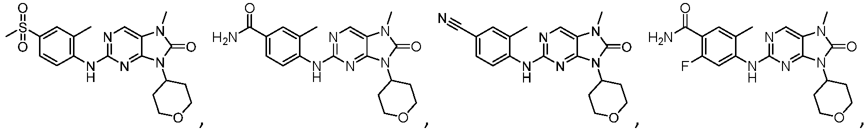

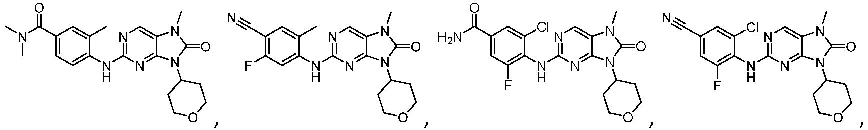

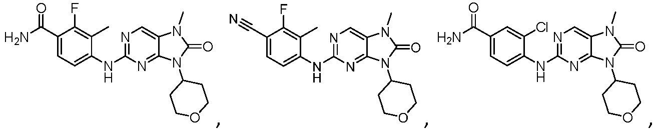

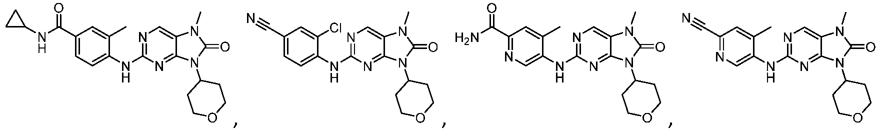

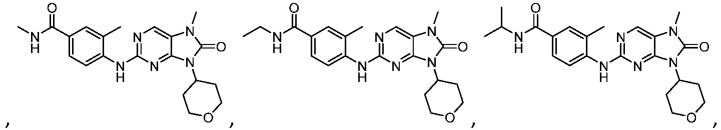

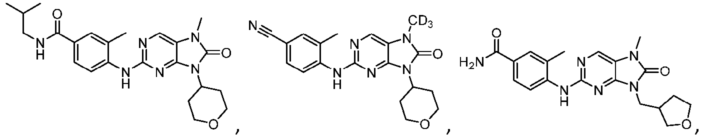

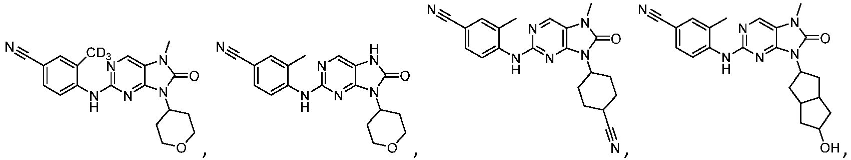

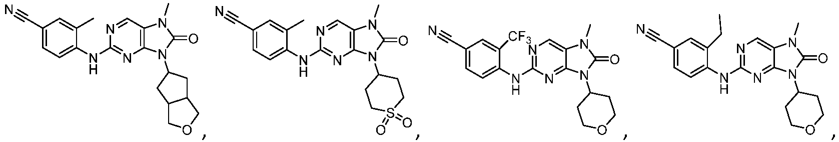

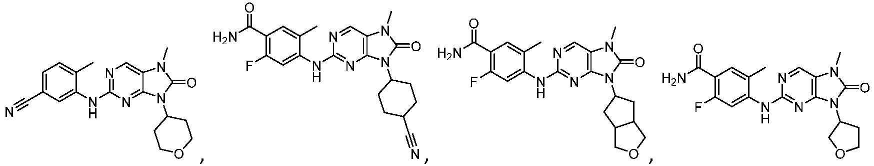

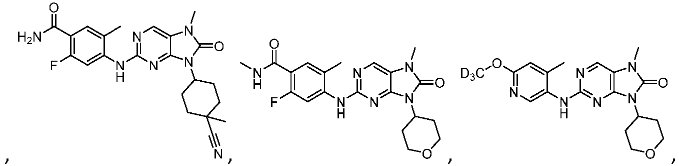

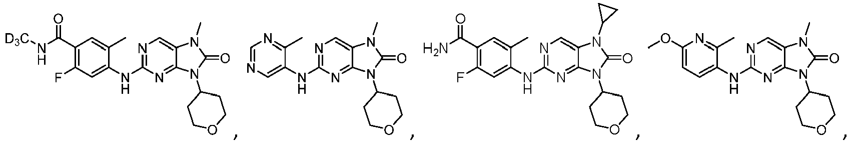

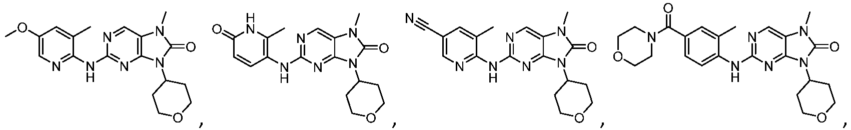

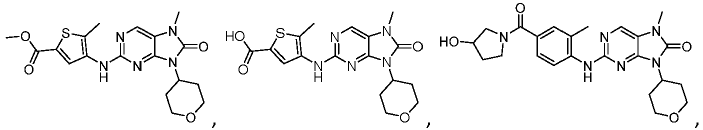

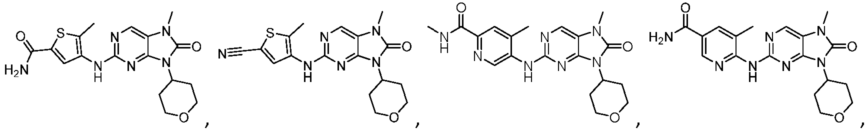

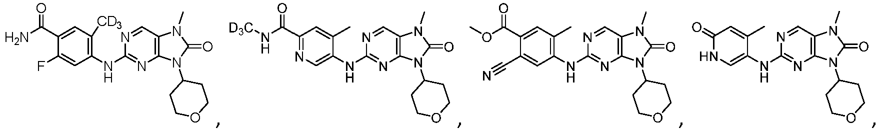

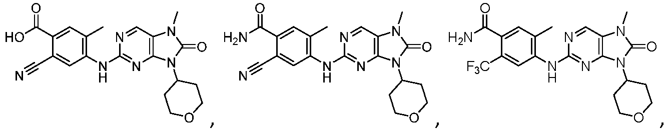

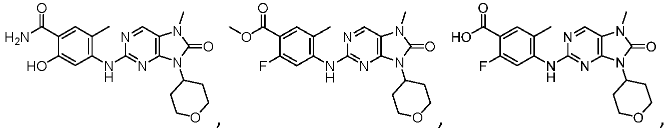

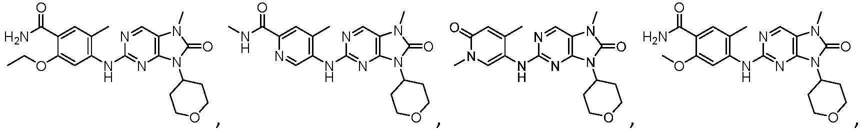

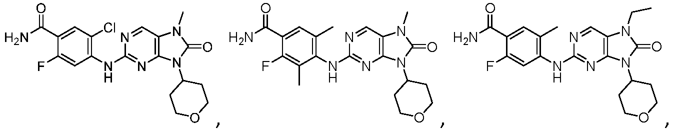

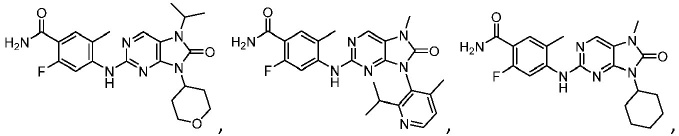

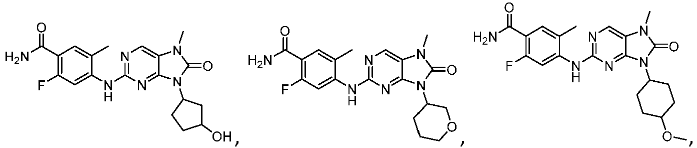

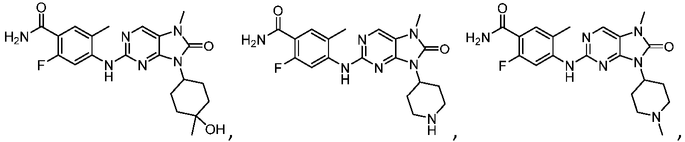

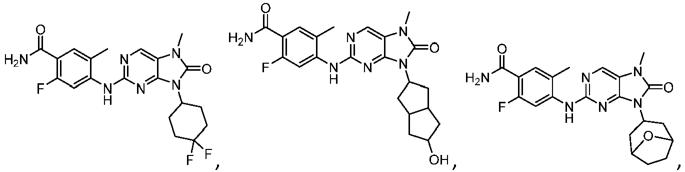

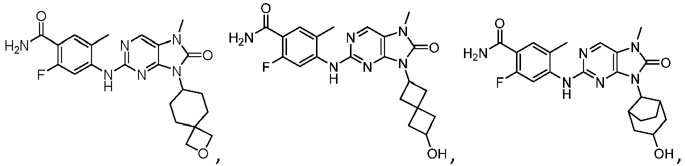

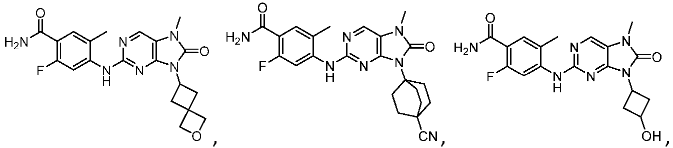

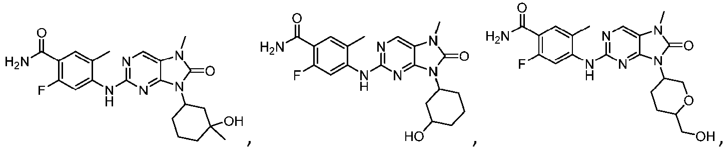

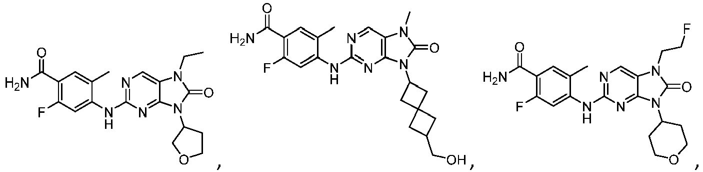

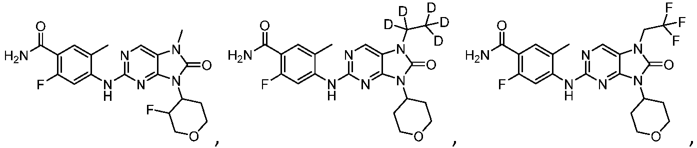

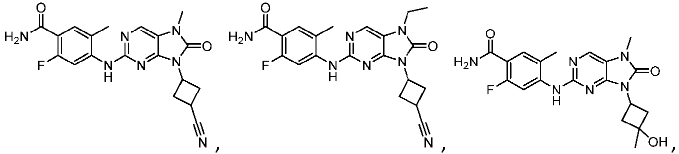

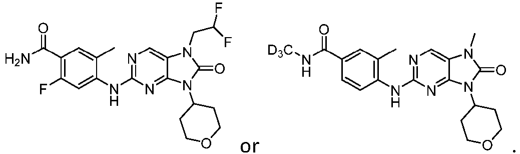

10. The pharmaceutical preparation according to any one of claims 1-9, wherein the pharmaceutical preparation comprises the compound of formula I with a specification of 1-5 mg, 5-10 mg, 10-15 mg, 15-20 mg, 20-25 mg, 25-30 mg, 30-35 mg, 35-40 mg, 40-50 mg, 50-60 mg, 60-70 mg, 70-80 mg, 80-90 mg, 90-100 mg, 100-150 mg, 150-200 mg, 200-250 mg, 250-300 mg, 300-350 mg, 350-400 mg, 400-450 mg, 450-500 mg, 500-600 mg, 600-700 mg, 700-800 mg, 800-900 mg or 900-1000 mg.

11. A pharmaceutical preparation of an imidazolinone compound, comprising an imidazolinone compound, or a stereoisomer, solvate, prodrug, metabolite, pharmaceutically acceptable salt or co-crystal thereof, wherein the imidazolinone compound is selected from the compound of formula I defined in any one of claims 1-9, and the content of the imidazolinone compound in the pharmaceutical preparation is 1-90 parts by weight.

12. The pharmaceutical preparation according to claim 11, wherein the content of the imidazolinone compound in the pharmaceutical preparation is 1-5 parts, 5-10 parts, 10-15 parts, 15-20 parts, 20-25 parts, 25-30 parts, 30-35 parts, 35-40 parts, 40-45 parts, 45-50 parts, 50-55 parts, 55-60 parts, 60-65 parts, 65-70 parts, 70-75 parts, 75-80 parts, 80-85 parts or 85-90 parts; and optionally further, the content of the imidazolinone compound in the pharmaceutical preparation is 27.8 parts or 41.7 parts.

13. The pharmaceutical preparation according to claim 11, wherein the dosage form of the pharmaceutical preparation is selected from a granule, a tablet, a capsule, a pill, or a micropellet.

14. The pharmaceutical preparation according to claim 11, wherein the pharmaceutical preparation further comprises one or more of a diluent, a disintegrant, and a glidant, and in the pharmaceutical preparation, in parts by weight, the content of the diluent is 10-85 parts; the content of the disintegrant is 3-70 parts; and the content of the glidant is 0.1-30 parts.

15. The pharmaceutical preparation according to claim 14, wherein in the pharmaceutical preparation, the content of the diluent is 10-15 parts, 15-20 parts, 20-25 parts, 25-30 parts, 30-35 parts, 35-40 parts, 40-45 parts, 45-50 parts, 50-55 parts, 55-60 parts, 60-65 parts, 65-70 parts, 70-75 parts, 75-80 parts, or 80-85 parts; the content of the disintegrant is 3-5 parts, 5-15 parts, 15-20 parts, 20-25 parts, 25-30 parts, 30-35 parts, 35-40 parts, 40-45 parts, 45-50 parts, 50-55 parts, 55-60 parts, 60-65 parts, or 65-70 parts; and the content of the glidant is 0.1-0.5 parts, 0.5-1 parts, 1-5 parts, 5-10 parts, 10-15 parts, 15-20 parts, 20-25 parts, or 25-30 parts; and optionally further, in the pharmaceutical preparation, the content of the diluent is 64.1 parts, 66.7 parts or 50 parts; the content of the disintegrant is 7 parts, 4.4 parts or 6.7 parts; and the content of the glidant is 1.6 parts or 1.1 parts.

16. The pharmaceutical preparation according to claim 14, wherein the diluent is selected from one or more of sucrose, xylitol, maltitol, lactitol, hydroxypropyl cellulose, hypromellose, sorbitol, glucose, fructose, corn starch, inorganic salts, microcrystalline cellulose, starch, mannitol, lactose and pregelatinized starch, preferably one or more of microcrystalline cellulose, mannitol and pregelatinized starch;
the disintegrant is selected from one or more of cross-linked polyvinylpyrrolidone, sodium starch glycolate, calcium carboxymethyl cellulose, potato starch, microcrystalline cellulose, low-substituted hydroxypropyl cellulose, croscarmellose sodium, crospovidone, sodium carboxymethyl starch and low-substituted hydroxypropyl cellulose; and
the glidant is selected from one or more of colloidal silica, silica, magnesium silicate, magnesium trisilicate, polysilicate, hydrated silica, calcium phosphate, and talc.

17. A preparation method for the pharmaceutical preparation of an imidazolinone compound according to any one of claims 1-16, comprising the following steps:
preparing a mixture of an imidazolinone compound, a disintegrant, and a diluent; and mixing a glidant with the mixture and then preparing same into a tablet, a capsule, or a granule.

18. The preparation method according to claim 17, wherein
after mixing a glidant with the mixture, the resulting mixture is placed in a granulator for granulation and then prepared into a tablet, a capsule, or a granule;
alternatively,
the mixture comprises 50-80 wt% of the prescription amount of the diluent; the mixture is placed in a mixer for mixing and then placed in a granulator for granulation to obtain mixture particles; and a glidant and the remaining prescription amount of the diluent are added to the mixture particles, and mixed in a mixer and then prepared into a tablet, a capsule, or a granule.

19. Use of the pharmaceutical preparation of an imidazolinone compound according to any one of claims 1-16 in the preparation of a drug for the treatment and/or prevention of a cancer.

20. Use of a pharmaceutical preparation in the preparation of a drug for the treatment and/or prevention of a cancer by administering to a subject a drug comprising the pharmaceutical preparation according to any one of claims 1-16; optionally further, the use involves administering the drug orally; and optionally further, the drug is administered orally 1-4 times per day.

21. A pharmaceutical preparation of an imidazolinone compound, comprising an imidazolinone compound, or a stereoisomer, solvate, prodrug, metabolite, pharmaceutically acceptable salt or co-crystal thereof, wherein the imidazolinone compound is selected from the compound of formula I defined in any one of claims 1-9, the compound of formula I is present in an amount to provide a daily dose of 1-1000 mg, and the daily dose is a single-dose or multi-dose.

22. The pharmaceutical preparation according to claim 21, wherein the imidazolinone compound is present in an amount to provide a daily dose of 1-10 mg, 10-20 mg, 20-50 mg, 50-100 mg, 100-150 mg, 150-200 mg, 200-250 mg, 250-300 mg, 300-350 mg, 350-400 mg, 400-450 mg, 450-500 mg, 500-600 mg, 600-700 mg, 700-800 mg, 800-900 mg or 900-1000 mg.

23. A pharmaceutical preparation of an imidazolinone compound, comprising an imidazolinone compound, or a stereoisomer, solvate, prodrug, metabolite, pharmaceutically acceptable salt or co-crystal thereof, wherein the imidazolinone compound is selected from the compound of formula I defined in any one of claims 1-9, and the compound of formula I is present in an amount to provide a daily dose of 1-1000 mg/60 kg.
